# EUROPEAN PATENT SPECIFICATION

(11) **EP 4 461 293 B1**
(45) Date of publication and mention of the grant of the patent: **19.08.2026**
(21) Application number: 23386035.2
(22) Date of filing: 12.05.2023
(51) Int. Cl.: A61K 31/282, A61K 33/243, A61P 35/00

(54) **BIOTIN RECEPTOR-BINDING ANTICANCER PLATINUM COMPOUNDS**
BIOTINREZEPTORBINDENDE ANTIKREBS-PLATINVERBINDUNGEN
COMPOSÉS ANTICANCÉREUX DU PLATINE SE LIANT AU RÉCEPTEUR DE LA BIOTINE

(43) Date of publication of application: 13.11.2024
(73) Proprietor: Biomedical Research Foundation Academy of Athens, 11527 Athens (GR)
(72) Inventor: Moos, Walter H., Oakland, CA 94611 (US); Steliou, Konstantinos, Natick, MA 01760 (US); Zamboni, Robert J., Beaconsfield, Quebec, H9W 5L4 (CA); Tamvakopoulos, Constantin, 14562 Kifissia (GR)
(74) Representative: Roukounas, Dimitrios

(56) References cited:
- CA-C- 1 282 058
- US-B1- 6 316 652

## Description

### FIELD OF THE INVENTION

The invention relates to the treatment of tumors that overexpress biotin receptors on their cell surface membranes.

### BACKGROUND OF THE INVENTION

Cancer remains a leading cause of death worldwide and the global cancer burden is on the increase as the population ages. Metastasized cancers are the most challenging tumor types to treat and despite many advances, metastatic disease remains essentially uncurable. Thus, effective therapies for these cancers represent an important and significant unmet medical need. Cisplatin, carboplatin and oxaliplatin are used in the treatment of a wide array of cancers and are the only platinum anticancer drugs that are currently approved for clinical use worldwide.

For some tumor types, cancer mortality has been steadily decreasing thanks to new pharmaceutical interventions and nonpharmaceutical innovations, such as improved screening, robotic surgery, advances in radiotherapy and other surgical techniques. However, the 5-year survival rate for many cancers, particularly for solid tumor types, has only modestly improved and this is largely due to better and earlier detection methods that increase the probability of diagnosing the cancer in a localized state when treatment is most effective.

Chemotherapy, surgery and radiotherapy are mainstay treatments but increasingly targeted biologics, engineered bacteria, engineered viruses and immuno-oncology checkpoint inhibitors are making their way into the arsenal of therapeutic options. In some cases, targeted biologics have significantly improved therapeutic outcomes. However, for a majority of cancer patients, immunotherapy with checkpoint inhibitors is not a therapeutic benefit for them. This is especially true in triple-negative breast cancer (TNBC), an exceptionally aggressive subtype accounting for 15-20% of all breast cancers. The outcomes for lung cancer (LC) and colorectal cancer (CRC) reflect similar modest response rates and thus far only a subset of patients have benefited from treatment with the checkpoint inhibitors clinically available.

### KAPA, DAPA and Clinically-Approved Platinum Drugs

Cisplatin, carboplatin and oxaliplatin, shown below, are used in the treatment of a wide array of cancers and are the only platinum anticancer drugs that are currently approved for clinical use worldwide.

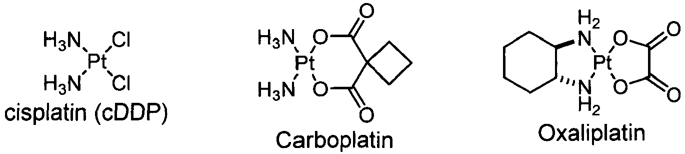

For a review of platinum compounds in cancer chemotherapy, see: Rottenberg, Disler & Perego [2021].

Cisplatin was approved by the US Food and Drug Administration (FDA) for the treatment of metastatic ovarian tumors [US4177263A, Anti-animal tumor method; US5562925A, Anti-tumor method] and ever since, it has become a staple in modern chemotherapy [Ghosh, 2019]. In addition to ovarian cancer, cisplatin is successful in the treatment of testicular cancer and is used in first-line chemotherapy against cancers of the lung, head-and-neck, esophagus, stomach, bladder, cervix and uterus. Platins are also used as second-line treatment against most other advanced cancer types such as cancers of the breast, colon, pancreas, liver, kidney and prostate as well as against glioblastomas, metastatic melanomas and peritoneal or pleural mesotheliomas [Ghosh, 2019].

Biomarkers provide useful information to help guide therapies, but they are not absolute determinants. For instance, despite a favorable initial response, BRCA-deficient cancer cells often become resistant to platinum drugs. However, when the platinum is given in combination with a poly(ADP-ribose)polymerase (PARP) inhibitor, in some cases, cancers with BRCA1/2 pathogenic variants are more effectively treated than using either drug alone [Rottenberg et al., 2021].

### Cisplatin (Carboplatin) Resistance

Intrinsic and/or acquired resistance hinder the effectiveness of platinum drugs in the treatment of cancer [Ghosh, 2019]. In clinical cancer settings, platinum-resistance refers to recurrence of the cancer within 6 months after the last dose of platinum-based chemotherapy was administered or biomarkers and/or tumor growth indicate the cancer continues to progress in spite of platinum therapy. Cisplatin resistance can arise from reduced platinum uptake, increased efflux, intracellular detoxification by glutathione (GSH), increased DNA repair, decreased mismatch repair, defective apoptosis, modulation of signaling pathways and/or the presence of quiescent non-cycling cells [Ghosh, 2019]. However, reduced cellular uptake and detoxification by GSH are the foremost pathways that enhance resistance to the platinum salts [Ghosh, 2019].

Platins have a remarkable and historical impact on cancer chemotherapy [Rottenberg et al., 2021]. More than half of all cancer chemotherapies since the clinical introduction of cisplatin forty plus years ago include a platin in the protocol [Ghosh, 2019]. However, despite intensive research, few new platinum compounds with adequate pharmacological advantages relative to cisplatin or carboplatin have emerged to warrant clinical development [Ghosh, 2019].

Unfortunately, multiple treatment cycles of cisplatin or carboplatin will induce drug resistance, which necessitates a change in the type of treatment and worse, premature cessation of therapy that frequently causes the cancer to relapse. Even with a favorable initial response, acquired resistance due to altered mutations and epigenetic events limits efficacy [Rottenberg et al., 2021]. Resistance to cisplatin or carboplatin developed during first-line treatment could also arise from alterations in DNA methylation resulting in the dysfunction of genes involved in cell-cell contact, in apoptosis and in several other pathways conferring a survival advantage to chemoresistant cells [Ghosh, 2019].

### Oxaliplatin for Treating Cisplatin-Resistant Cancers

Platinum-based chemotherapy is laden with severe adverse reactions including nephrotoxicity (irreversible), gastrointestinal toxicity (emetogenesis), peripheral neuropathy (has potential to become irreversible), bone marrow suppression, asthenia, ototoxicity (irreversible), and partial loss of vision due to retinal detachment, thinning of the optic nerve fibers, changes in the electroretinogram, occlusion of the middle retinal, and cilioretinal arteries.

Oxaliplatin, (*trans-R,R*-1,2-diaminocyclohexane)oxalate platinum(II), is widely regarded as being active in cisplatin-resistant cancers and is the first-line treatment for colorectal cancer - carboplatin and cisplatin are less effective [Ghosh, 2019]. The enhanced therapeutic action of oxaliplatin over cisplatin and carboplatin is attributed to its diaminocyclohexyl ligand (DACH). It is theorized that the molecular shape and lipophilic nature of DACH allow oxaliplatin to form platinum-DNA adducts with less structural distortion which escape the vigilant DNA excision repair enzymes that capture and remove DNA-bound cisplatin and carboplatin-type adducts.

Platinum(II) salts are square-planar and are most effective as a cancer drug when their labile leaving groups (anionic radicals such as chlorides, oxyl, etc.) are replaced with molecules of water, desirably after entering the cancer cell, to form a positively charged platinum hydrate. This positively charged hydrated platinum binds to form cell-toxic adducts with DNA, RNA and proteins in the cell. DNA of purine bases on the N7 loci is critical in platinum antitumor drug targets leading to tumor cell cycle arrest or apoptosis.

Recent studies credit the therapeutic activity of oxaliplatin against colorectal cancer to its enhanced uptake over cisplatin or carboplatin in tumors that organic cation transporters (OCTs) are highly expressed [Rottenberg et al., 2021]. An additional factor that also may be contributing to the antitumor activity of oxaliplatin is the influence of commensal microbiota in the gut [Rottenberg et al., 2021].

### Ferroptosis

Iron plays a critical role in maintaining homeostasis in cells. It is required for cell growth and for essential cellular functions, including mitochondrial respiration, DNA/RNA synthesis, enzyme function, and oxygen transport [Rishi, Huang, & Subramaniam, 2021]. The ability of iron to readily undergo redox reactions is central to all these functions and cellular iron levels in normal tissue are tightly regulated [Rishi et al., 2021]. Ferroptosis is an iron-dependent cell death, which is different from apoptosis, necrosis, autophagy, and other forms of cell death. Dysfunctional and damaged mitochondria turn cytotoxic by releasing cell death factors and by producing an excessive amount of reactive oxygen species (ROS). ROS can damage DNA, proteins and/or react with polyunsaturated fatty acids (catalyzed by the active participation of labile iron) resulting in lipid peroxidation, which ultimately lead to cell death and tissue damage [Rishi et al., 2021].

To retain more iron for their growth and metabolic needs, cancer cells develop mechanisms that increase their iron uptake and decrease iron export. In normal cells, the accumulating excess labile iron (Fe²⁺) in mitochondria, the major cellular site of iron utilization, generates ROS, which activates the ferroptotic machinery resulting in cell death. However, cancer cells have been shown to survive this by developing mechanisms to circumvent ferroptosis, such as by upregulation of transcription factor nuclear factor erythroid 2 (NFE2)-related factor 2 (Nrf2) [Rishi et al., 2021].

Thus, drugs that preferentially target and disrupt cancer cell mitochondrial integrity can cause an increase in the production of ROS and, thereby, enhance the chemotherapeutic effect of these drugs in the treatment of tumors by activating ferroptosis.

### DAPAplatins Targeting Tissue-Agnostic Biotin Receptor-Positive Tumors

Malignant dividing cells require higher vitamin uptake (e.g., B₁₂, folic acid, biotin, riboflavin, etc.) to sustain their rapid growth. To facilitate the tumor's uptake of these vitamins, some tumor types, such as breast cancer and lung cancer cells, overexpress sodium-dependent multivitamin transporters (SMVT) and the vitamin receptors on their cell surface membrane. Russel-Jones and McEwan [US20060127310A1, Amplification of biotin-mediated targeting] teaches how their invention, which relates to enhanced substance delivery via the biotin uptake system, could be used in the treatment and diagnosis of cancer by means of a biotin-active substance-polymer conjugate or a biotin-nanoparticle conjugate. However, not all human cancer types overexpress biotin receptors on their cell surface membrane. To help distinguish tumor types with enhanced biotin receptor expression (referred to as biotin receptor-positive cancer cells) from biotin negative expression cancers, Ren et al. [2015] studied the relative uptake of biotin in numerous cancer cell lines (listed in Table 1 below) as either having positive or negative biotin receptor expression. In addition, it has been demonstrated in the prior art that biotin receptor-positive tumors may be ascertained by PET-MRI using a ⁶⁸Ga-IO@biotin-TSCDex nanoprobe [Gholipour, Akhlaghi, Mokhtari Kheirabadi, Geramifar, & Beiki, 2020]. Additionally, small-molecule fluorescent probes have also been used to distinguish between biotin receptor-positive cancer cells/tissues and biotin receptor-negative normal cells/tissues as described in Wang et al. 2023.

**Table 1. Various human and rodent cell lines with positive or negative biotin receptor expression [Ren et al., 2015].**

| **Biotin Positive Cell Lines** | **Origin** | **Degree of Biotin (+/-) expression** |
|---|---|---|
| MCF-7 | Human breast cancer cell | +++ |
| MCF-10A-H-RasV12 | Genetically modified human breast cancer cell | ++ |
| BT-20 | Human breast cancer cell | ++ |
| LCC6-WT | Human breast cancer cell | ++ |
| LCC6-MDR | Human breast cancer cell | + |
| MDA-MB 231 | Human breast cancer cell | ++ |
| SkBr3 | Human breast cancer cell | ++ |
| 4T1 | Human breast cancer cell | +++ |
| A549 | Human lung cancer cell | +++ |
| HepG2 | Human hepatic carcinoma cell | +++ |
| HeLa | Human cervical cancer cell | +++ |
| MX-1 | Human duct carcinoma | ++ |
| OVCAR-3 | Human ovarian cancer cell | ++ |
| OV2008 | Human ovarian cancer cell | ++ |
| Colo-26 | Human colorectal adenocarcinoma cell | +++ |
| MMT06056 | Human breast cancer cell | +++ |
| ID8 | Mouse ovarian cancer cell | ++ |
| M109 | Mouse lung carcinoma | +++ |
| JC | Mouse breast adenocarcinoma cell | +++ |
| L1219FR | Mouse lymphocytic leukemia cell | +++ |
| RENCA | Mouse renal adenocarcinoma cell | +++ |
| RD0995 | Mouse renal cell | +++ |
| P815 | Mouse mastocytoma | +++ |
| 4T1 | Mouse breast cancer cell | +++ |

| **Biotin Negative Cell Lines** | | |
|---|---|---|
| HEK-293 | Human embryonic kidney cell | - |
| HCT-116 | Human colon cancer cell | - |
| MCF-10A-Vector | Genetically modified human breast cancer cell | - |
| WI38 | Normal human lung fibroblast | - |
| BW5147 | Mouse lymphoma T-cell | - |
| B16 | Mouse skin melanoma cell | - |
| NIH3T3 | Mouse embryonic fibroblast | - |
| LL-2 | Mouse Lewis lung carcinoma cell | - |
| CHO | Noncancerous Chinese hamster ovarian cell | - |
| L1210 | Mouse lymphocytic leukemia cell | - |

D-(+)-Biotin (vitamin H or B₇) is an essential cofactor in carboxylation transfers and indispensable to all living organisms [Khoury, Deo, & Aldrich-Wright, 2020]. It is biosynthesized in bacteria, plants and some fungi via D-(+)-desthiobiotin, (7*R*,8*S*)-7,8-diaminononanoic acid, commonly known as 7,8-diaminopelargonic acid (DAPA), and its immediate biological precursor, (8*S*)-8-amino-7-oxononanoic acid, also referred to as 7-keto-8-aminopelargonic acid (KAPA) (shown below).

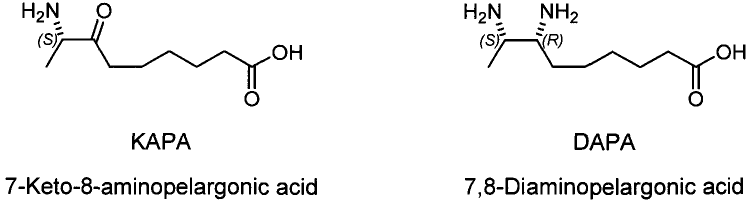

In mammals, biotin also regulates gene expression through biotinylation and activation of enzymatic activity and signal transduction pathways. Notably, higher organisms lack the ability of commensal bacteria to synthesize biotin and thus depend primarily on exogenous sources (such as diet and/or supplements) to satisfy their vitamin needs [Azhar, Booker, & Polyak, 2015; Zempleni, Wijeratne, & Hassan, 2009].

Mitochondria are vital cellular organelles that play critical roles in cancer progression and metastasis, including tumor biology and metabolism. Tumor heterogeneity and therapeutic resistance are mainly driven by epigenetic alterations [Rottenberg et al., 2021]. Biotin, folate and B₁₂ are well-known cancer-specific vitamins [Ren et al., 2015]. Tumors have a high demand for biotin, which is required to sustain their rapid growth. Many different tumor types, such as breast cancer, lung cancer and ovarian cancer overexpress biotin receptors on their cell surface membranes. Other cell types, such as human colon cancer (HCT-116), human lung fibroblasts (WI38) and human embryonic kidney cells (HEK-293) do not [Khoury et al., 2020; Ren et al., 2015]. Human cells rely on the sodium-dependent multivitamin transporter (SMVT) and/or monocarboxylate transporter 1 to acquire biotin by active transport [Azhar et al., 2015; Khoury et al., 2020; Zempleni et al., 2009] and, in cooperation with other biotin-binding proteins, partition biotin to compartments (cytoplasm, mitochondria and nuclei) involved in biotin signaling [Zempleni et al., 2009]. For example, it has been demonstrated that the vast majority of biotin in rat liver localizes to mitochondria and cytoplasm, whereas only a small fraction localizes to microsomes. The relative enrichment of biotin in mitochondria and cytoplasm is consistent with the role of biotin as a coenzyme for carboxylases in these compartments. [Zempleni et al., 2009].

A small (0.7%) but qualitatively important fraction of biotin also localizes to the cell nucleus and this increases to about 1% of total biotin in response to proliferation [Zempleni et al., 2009].

US6316652B1 discloses cisplatin derivatives that are targeted for entry into the mitochondria and are useful as anticancer agents.

### SUMMARY OF THE INVENTION

The present invention relates to compounds exhibiting anticancer activity.

Although the compounds of the present invention comprise platinum, their cellular uptake differs from the cellular uptake of other platinum compounds known in the art. Namely, it is known that cellular uptake of cisplatin, carboplatin, and oxaliplatin in vivo occurs primarily by passive diffusion and, to some degree, also assisted by organic cation and copper transporters [Johnstone, Suntharalingam, & Lippard, 2016]. Recent studies suggest that about half of cisplatin and carboplatin but not oxaliplatin may enter cells through the widely expressed volume-regulated anion channel (VRAC) [Rottenberg et al., 2021]. On the other hand, by utilizing the mammalian biotin uptake and transport machinery, the compounds of the present invention are transported into the cancer cells where they exert their anticancer activity.

The present invention provides therefore, a compound of Formula (I), or a pharmaceutically acceptable salt thereof, 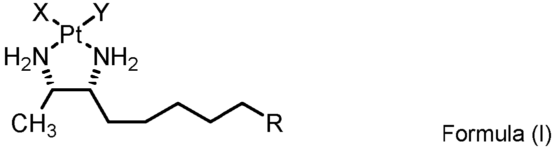 wherein
R is COOH,
   X and Y are the same or different and each is selected from CI, Br, I, or, X and Y are joined to form a ligand 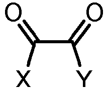
wherein each one of X and Y is O,
for use in the treatment of cancer, wherein the cancer cells overexpress biotin receptors on their cell surface membranes.

The present invention provides also a pharmaceutical composition comprising a compound of Formula (I), or a pharmaceutically acceptable salt thereof, and a pharmaceutically acceptable carrier, for use in the treatment of cancer, wherein the cancer cells overexpress biotin receptors on their cell surface membranes.

### BRIEF DESCRIPTION OF THE DRAWINGS

Figure 1 shows viability of human MSCs, derived from fetal umbilical cord matrix (Wharton's jelly), after 48 h growth in the presence of cisplatin, carboplatin and compound of the present invention PMX114, each independently determined by MTT assay.
Figure 2 shows the CyTOF Pt195 signals in MDA-MB-231 (left panel) & A549 (right panel) cell lines each treated separately with 50 µM of carboplatin and 50 µM of the compound of the present invention PMX114.
Figure 3 shows the CyTOF Pt195 signals in SkBr3 cell line preincubated with 100 µM biotin or not and then treated separately with 50 µM of carboplatin and 50 µM of the compound of the present invention PMX114.
Figure 4 shows the average concentration of carboplatin and the compound of the present invention PMX 114 in mouse plasma following intraperitoneal administration: carboplatin at 60 mg/kg; PMX114 at 75 mg/kg.

### DETAILED DESCRIPTION OF THE INVENTION

The present invention provides a compound of Formula (I), or a pharmaceutically acceptable salt thereof, 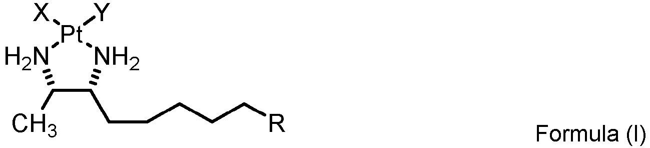 wherein
R is COOH,
   X and Y are the same or different and each is selected from CI, Br, I, or, X and Y are joined to form a ligand 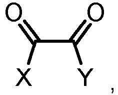
wherein each one of X and Y is O,
for use in the treatment of cancer, wherein the cancer cells overexpress biotin receptors on their cell surface membranes.

According to a preferred embodiment, the compound of Formula (I) has the following structure and is referred to hereinafter as PMX114:

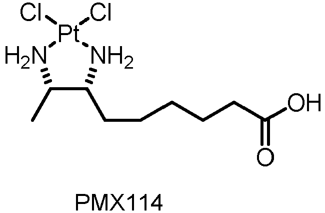

According to another preferred embodiment, the compound of Formula (I) has the following structure and is referred to hereinafter as PMX118:

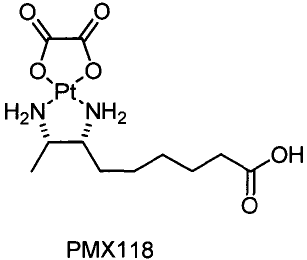

The term "biotin receptors" means cell surface membrane receptors that bind biotin (or an analogue thereof, such as diaminobiotin or desthiobiotin), in particular sodium-dependent multivitamin transporter (SMVT), and/or monocarboxylate transporter 1.

In platinum-based chemotherapies, resistance to the platinum drug, and/or toxic platinum-associated side effects, owing to indiscriminate cellular uptake of the platinum complex from blood plasma, hinder the effectiveness of the treatment, often leading to premature cessation of therapy that frequently causes the cancer to relapse.

The present invention is directed to the treatment of tissue-agnostic cancer types that overexpress biotin receptors on their cell surface membrane. Thus, the invention is not restricted to a specific tissue cancer type. Examples of cancer types which can be treated with the present invention include, breast cancer, cervical cancer, colorectal adenocarcinoma, hepatic carcinoma, human duct carcinoma, leukemia, lung cancer, mastocytoma and ovarian cancer. Preferably, the cancer is selected from breast cancer, cervical cancer, colorectal adenocarcinoma, hepatic carcinoma, leukemia, lung cancer, mastocytoma and ovarian cancer. More preferably, the cancer is selected from breast cancer, colorectal adenocarcinoma and lung cancer.

The compounds of the present invention can be prepared from 7,8-Diaminopelargonic acid (DAPA) as depicted in Scheme 1 shown below:

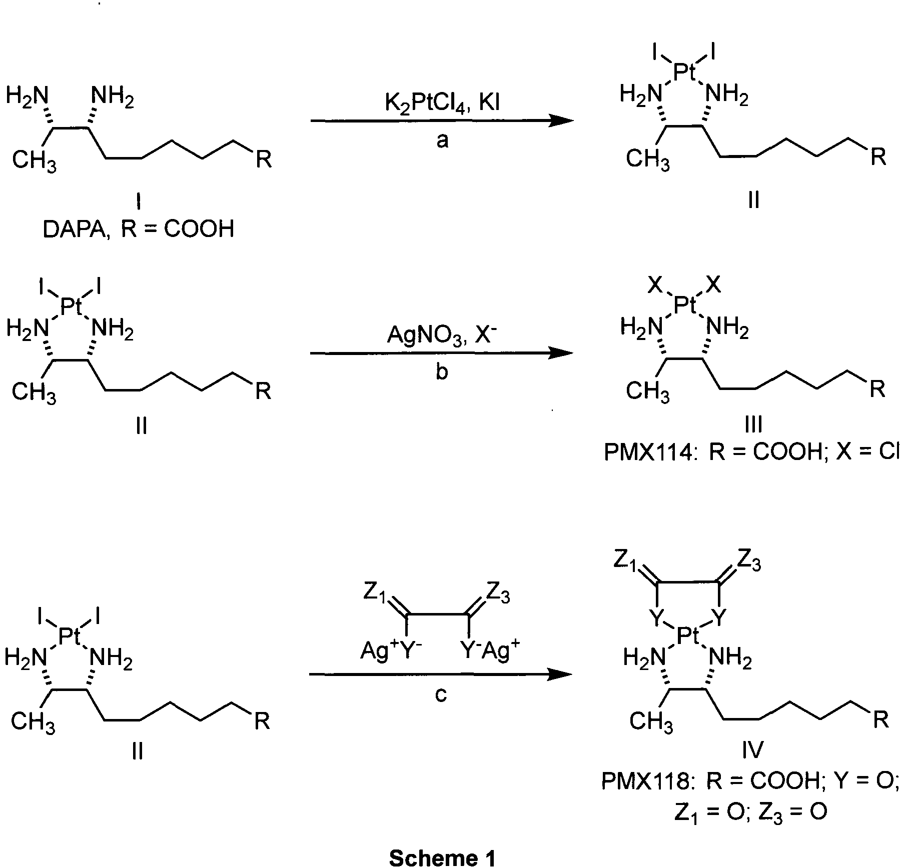

Compound II in Scheme 1 above can be prepared, for example, (a) by reacting compound I with K₂PtCl₄ and a suitable halide salt (in Scheme 1, KI is exemplified) in a suitable solvent, such as water, at 25 °C in the dark. Compound III can be prepared, for example, (b) by reacting compound II with AgNO₃ in aqueous HCl at elevated temperature, for example at 45 °C in the dark. Compound IV can be prepared, for example, (c) by reacting compound II with the appropriate ligand (in Scheme 1 with silver oxalate) in a suitable solvent, for example water, at elevated temperature, for example at 60 °C, in the dark.

The compounds of the present invention can form pharmaceutically acceptable salts with an inorganic or an organic acid. Examples of inorganic acid addition salts include hydrochloride, hydrobromide, sulfate, nitrate, and phosphate. Examples of organic acid addition salts include acetate, lactate, citrate, tartrate, succinate, maleate and fumarate. The pharmaceutically acceptable salts can be prepared following processes well known in the art.

Preferably, the compound of Formula (I), or a pharmaceutically acceptable salt thereof, for use as defined above, is administered to a subject, preferably a human. Preferably, the compound of Formula (I) or a pharmaceutically acceptable salt thereof, is administered as a composition comprising the compound of Formula (I), or a pharmaceutically acceptable salt thereof, and a pharmaceutically acceptable carrier. The present invention provides also a pharmaceutical composition comprising a compound of Formula (I), or a pharmaceutically acceptable salt thereof, and a pharmaceutically acceptable carrier, for use in the treatment of cancer, wherein the cancer cells overexpress biotin receptors on their surface membranes.

The composition may be formulated, for example, for parenteral, intramuscular, intracerebral, intravenous, intraperitoneal, subcutaneous, transdermal/intradermal, intratumoural, intrarectal, oral, or inhalation administration. The composition may have different forms, such as solid or liquid forms, for example it can have the form of powder, tablet, capsule, suppository, solution, suspension, emulsion, gel, cream, ointment, spray, or transdermal patch. The composition typically comprises a compound of Formula (I), or a pharmaceutically acceptable salt thereof as active ingredient and a pharmaceutically acceptable carrier. The pharmaceutically acceptable carrier may comprise one or more excipients, such as solvents, buffering agents, emulsifying agents, preservatives, antioxidants, diluents, binders, disintegrants, lubricants and the like, which are well known in the art.

Examples of solvents include saline, water, phosphate buffered saline, ethanol, isopropyl alcohol and mixtures thereof.

Examples of buffering agents include citric acid monohydrate, acetic acid, sodium acetate and sodium hydrogen phosphate.

Examples of emulsifying agents include sodium lauryl sulfate, lecithin, polysorbate and sorbitan monooleate.

Examples of preservatives include sodium benzoate, benzyl alcohol and parahydroxy benzoic acids and their alkyl esters.

Examples of antioxidants include sodium metabisulfite, butylated hydroxyanisole, butylated hydroxytoluene and ascorbic acid.

Examples of diluents include lactose, sucrose, dextrose, mannitol and sorbitol.

Examples of binders include hydroxypropylcellulose, hydroxyethylcellulose, dextrose, xylitol, polyvinylpyrrolidone and polyethylene glycol.

Examples of disintegrants include starch, clay, cellulose, gum, polyvinyl polypyrrolidone and cross-linked polyvinyl polypyrrolidone.

Examples of lubricants include magnesium stearate, aluminium stearate, magnesium carbonate, magnesium oxide and sodium benzoate,

The compounds of the present invention may be administered orally, transdermally, intravenously, intranasally, rectally or by any means that delivers an effective amount of the agent to the tissue or site to be treated. Suitable dosages are those that achieve the desired endpoint. It will be appreciated that different dosages may be required for treating different neoplastic disorders. An effective amount of an agent is that amount which causes a significant decrease in neoplastic cell count, growth, or size.

Those having ordinary skill in the art will be able to ascertain the most effective dose and times for administering the compounds of the present invention, considering route of delivery, metabolism of the compound and other pharmacokinetic parameters such as volume of distribution, clearance, age of the subject, etc.

Typical patient-specific dosages can range from 100 mg/m² to 800 mg/m², preferably, 200 mg/m² to 700 mg/m², and most preferably, 300 mg/m² to 500 mg/m². When administered by infusion, the active ingredient can typically be administered at a rate of 1 mg/mL/min to 8 mg/mL/min, preferably, 2 mg/mL/min to 7 mg/mL/min, more preferably, 3 mg/mL/min to 5 mg/mL/min. The administration can be carried out, for example, at least once daily, or every other day, or weekly, or every other week.

### EXAMPLES

### Example Experiments and Empirical Data

To demonstrate the merits and use of the present invention, a series of planned experiments and empirical data are presented below.

Samples of cisplatin, carboplatin and oxaliplatin were purchased from commercial sources. DAPAplatins^{™} PMX114 and PMX118 were custom synthesized by Zamboni Chem Solutions (Beaconsfield, Quebec, Canada) according to the general procedures and methods described in the literature [Jolley, Yanovsky, Kelland, & Nolan, 2001; Moradell et al., 2003; Wilson & Lippard, 2014] as detailed in Example 1 and in Example 2 below, and were characterized as analytically pure by NMR and LC-MS.

### Example 1

Preparation of (7*R*,8*S*)-7,8-diaminononanoic acid dichloroplatinum(II) (PMX114).

(7*R*,8*S*)-7,8-Diaminononanoic acid diiodoplatinum(II). An aqueous solution of K₂PtCl₄ (415 mg, 1 mmol) mixed with KI (1.0 g, 6.02 mmol) in water (5 mL) was stirred for 30 min at ambient temperature in the dark. To this was added an aqueous solution (minimum amount of water) of 286.3 mg of DAPA sulfate (1 mmol) pre-neutralized with 138.2 mg solid K₂CO₃ (1 mmol). A dark olive colored precipitate formed gradually. The mixture was left to stir at rt in the dark for 24 h. The precipitate was collected by filtration, washed with water and allowed to dry under air to give 573 mg (90%) of crude diiodoplatinum(II) complex.

A suspension of the crude diiodoplatinum(II) complex (560 mg, 0.88 mmol) in an aqueous (15 mL) solution of AgNO₃ (329 mg, 1.94 mmol) was stirred at 45 °C in the dark overnight. The resulting silver iodide was collected by filtration and washed with cold water. To the combined filtrate and washings was added a few drops of 1 N HCl to precipitate any remaining silver ions. The volume of the filtrate was reduced to 1.0 mL by evaporation at 35 °C and 0.5 mL (6 mmol) of 12 N HCl was added. The mixture was warmed at 45 °C for 15 min and then cooled in an ice-water bath to crystallize out the pale yellow platinated diaminodichloride. The crystals were collected by filtration, washed with cold 1 N HCl and dried in air (by suction) to afford 280 mg (70%) of PMX114 as a yellow powder: mp 215-218 dec.; [α]²³_{D} = +64.0° (c 0.1, DMF); IR (KBr) 3244, 3175, 3105, 2942, 2865, 1709, 1598, 1414, 1295, 1215, 1115, 922, 608 cm⁻¹; ¹H NMR (400 MHz, DMF-d₇) δ 12.23 (s, 1H, COOH), 5.58 (br d, 1H, *J* = 10.4 Hz, A-NH₂), 5.52 (br d, 1H, *J* = 10.4 Hz, B-NH₂), 5.13 (br d, 1H, *J* = 10.4 Hz, B-NH₂), 5.03 (dd, 1H, *J =* 10.4, 7.6 Hz), 3.02-2.95 (m, 2H, C7-H, C8-H), 2.29 (t, 2H, *J* = 7.2 Hz, C2-2H), 1.71-1.62 (m, 2H, C6-2H), 1.58 (quintet, 2H, *J* = 7.6 Hz, C3-2H), 1.50-1.38 (m, 4H, C4-2H, C5-2H), 1.33 (d, 3H, *J* = 6.8 Hz, C9-3H); ¹³C NMR (67.8 MHz, DMF-d₇) δ 177.4, 65.7, 60.0, 36.6, 31.9, 29.5, 27.7, 27.6, 15.0; Solubility: good in DMF.

### Example 2

### Preparation of (7R,8S)-7,8-diaminononanoic acid oxalatoplatinum(ll) (PMX 118).

To a suspension of the crude diiodoplatinum(II) complex of Example 1 (1.26 g, 1.98 mmol) in H₂O (100 mL) was added 1 N NaOH (2.0 mL, 2.0 mmol), and the mixture stirred for 30 min at rt to obtain a clear brown solution. Then, 607.5 mg of disilver oxalate (2.0 mmol) was added and the reaction mixture left to stir at 60 °C in the dark overnight. The resulting precipitate was collected by filtration. The filtrate was neutralized (slightly acidic) with oxalic acid and the solvent removed under vacuum using a high efficiency rotary to give PMX118 as a pale white solid. Recrystallized from water and characterized by ¹H NMR (400 MHz, DMF-d₇) δ 12.0 (s, 1H, COOH), 6.3 (br d, 1H, *J =* 10.4 Hz, A-NH₂), 5.8 (br d, 1H, *J* = 10.4 Hz, B-NH₂), 5.6 (br d, 1H, *J* = 10.4 Hz, B-NH₂), 5.3 (dd, 1H, *J* = 10.4, 7.6 Hz), 2.6-2.4 (m, 2H, C7-H, C8-H), 2.2 (t, 2H, *J* = 7.2 Hz, C2-2H), 1.7-1.6 (m, 2H, C6-2H), 1.6 (quintet, 2H, *J* = 7.6 Hz, C3-2H), 1.5-1.4 (m, 4H, C4-2H, C5-2H), 1.3 (d, 3H, *J* = 6.8 Hz, C9-3H).

### Example 3

### Assessing Toxicity to Normal Cells:

Mesenchymal stem cells (MSCs) represent normal somatic cells with an ability to self-renew and to differentiate towards a variety of specialized cell types through a combination of symmetric and asymmetric divisions [Christodoulou et al., 2022]. Characteristics that make MSCs an ideal cell type to assess in vitro platin toxicity to normal cells [Dimitrijević Stojanović et al., 2022]. Thus, human mesenchymal stem cells were extracted from Wharton's jelly (matrix) of the human fetal umbilical cord, also known as Wharton's Jelly Mesenchymal Stem cells (WJ-MSCs), from full-term pregnancies, as described in Christodoulou et al. [2022] and Goulielmaki et al. [2019].

### Protocol

WJ-MSCs up to seventh passage were used for this in vitro assay (b22 population doublings). The mean population doubling time (PDT) was 32+/- hours and cells maintained a consistent MSC immunophenotypic profile. Cells were plated in 75 cm² flasks and cultivated in growth media composed of DMEM/F12 (with 3.5 g/L glucose, ultraglutamine I, and Na pyruvate from Lonza (Basel, Switzerland)) supplemented with 10% FBS, 15 mM HEPES, 1x nonessential amino acids and 1% penicillin/streptomycin (all from Invitrogen (Waltham, Massachusetts, USA)). The cells were incubated at 37 °C in an environment of 5% CO₂. Medium changes took place every 3-4 days till 70% to 80% confluence. For new cell passages, 0.05% trypsin-EDTA solution (Invitrogen) was used, and the cells were then resuspended at a density of 4000 cells per cm² in 75 cm² flasks.

### MTT [3-(4,5-dimethylthiazol-2-yl)-2,5-diphenyltetrazolium bromide] Viability Assays

Cell viability of (WJ-MSCs) was determined colorimetrically in a 96-well plate assay, using the tetrazolium compound MTS (3-[4,5,dimethylthiazol-2-yl]-5-[3-carboxymethoxy-phenyl]-2-[4-sulfophenyl]-2H-tetrazolium, inner salt), which is bioreduced by cells into a colored formazan product that is soluble in tissue culture medium. NADPH or NADH, which are products of dehydrogenase enzymes in metabolically active (viable) cells, appear to be responsible for this conversion.

### Protocol

At a concentration of 3.5x10² cells/200 µL culture medium/well (37 °C, 5% CO₂), WJ-MSCs were plated into 96-well microtiter plates. The medium was removed after 48 hours of incubation, and the cells were then treated with up to eight different drug concentrations, diluted in 200 µL of growth medium, in each well. In control wells only growth media was added, and the cells were incubated for an additional 48 hours. The cells were then washed with 150 µL/well of prewarmed PBS after the drug/medium solutions had been removed. After that, in each well was added 20 µL of CellTiter 96 AQueous One Solution Reagent (Promega, Madison, Wisconsin, USA) and 100 µL of culture media (without phenol red, L-glutamine, or HEPES). Following that, cells were cultured for 3 hours. At the end, the absorbance was measured at 490nm (with 650nm as a reference wavelength) in a microplate reader (monochromator) [Monochro-mator Microplate Reader safire2 TECAN AUSTRIA GMBH/Measurement parameter Editor Magellan (version 6)].

### Results

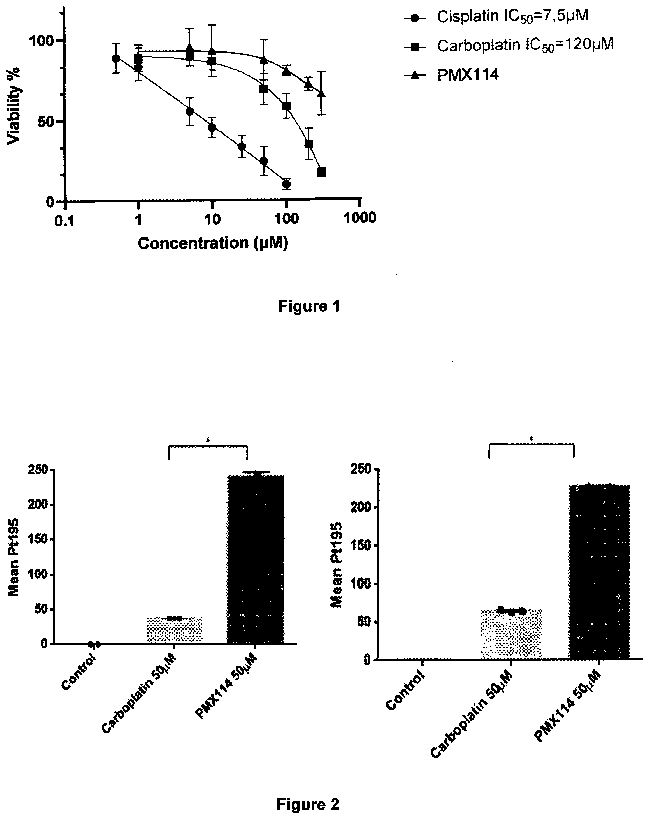

Less than 50% viability is respectively reached at approximately 5 µM for cisplatin (highly toxic) and at 100 µM for carboplatin (Figure1), a similar level of toxicity for PMX114 was not observed even in excess of 300 µM.

### Example 4

A head-to-head comparative analysis of the IC₅₀ (the concentration of a drug or inhibitor needed to inhibit a biological process or response by 50%) values for PMX114 and carboplatin in an MTT viability assay (the protocol described in Example 3), using SkBr3 human breast cancer cells, which overexpress biotin receptors on their cell surface membranes:

### Results:

Table 2a shows that IC₅₀ values for carboplatin are insensitive to the presence of co-administration of D-(+)-biotin, whereas co-administration of D-(+)-biotin significantly inhibits the cellular uptake of PMX114.

**Table 2a. In vitro activities (IC₅₀) against (NIH) SkBr3 with and without addition of 100 µM D-(+)-biotin.**

| **Platin (µM, 72h)** | **SkBr3 IC₅₀** | **SkBr3 and biotin IC₅₀** |
|---|---|---|
| **Carboplatin** | 9 ± 1 | 9 ± 1 |
| **PMX114** | 12 ± 2 | 40 ± 12 |

Table 2b shows that IC₅₀ values for cisplatin and carboplatin are insensitive to the presence of co-administration of D-(+)-biotin, whereas co-administration of D-(+)-biotin significantly inhibits the cellular uptake of PMX114 and PMX118.

**Table 2b. In vitro activities (IC₅₀) against MCF-7 with and without addition of 10 µM D-(+)- Biotin**

| **Platin (µM, 72h)** | **MCF-7 IC₅₀** | **MCF-7 and biotin IC₅₀** |
|---|---|---|
| **Cisplatin** | 9 | 12 |
| **Carboplatin** | 101 | 94 |
| **PMX114** | 90 | 150 |
| **PMX118** | 133 | 290 |

Carnitine esters of platinum (US6316652B1), referred to as mitoplatins, were shown by Gang Yao [Yao, 1999] to be inactive as anticancer drugs in vivo.

### Example 5

IC₅₀ values for each of the cell lines used were obtained using the MTT assay protocol described in Example 3.

### Results

Table 3a presents the IC₅₀ values for HCT-116 (human colorectal carcinoma cells that do not overexpress their biotin receptors [Ren et al., 2015]), and Table 3b, the IC₅₀ values for SKBr3 (human breast cancer cell line that overexpresses the HER2 (Neu/ErbB-2) gene product), MDA-MB-231 (epithelial, human metastatic mammary adenocarcinoma TNBC cells), and MCF-7 (human breast cancer cell line with estrogen, progesterone and glucocorticoid receptors) are cell lines that overexpress on their cell surface membranes biotin receptors (biotin receptor-positive) [Ren et al., 2015].

**Table 3a. Platin IC₅₀ values in human colon cancer cells.**

| **Platin (µM, 72h)** | **HCT-116 IC₅₀** |
|---|---|
| **Cisplatin** | 7, 8 |
| **Carboplatin** | 142, 155 |
| **PMX114** | 135, 148 |
| **PMX118** | 225, >250 |

**Table 3b. Platin IC₅₀ values in human breast cancer cells.**

| **Platin (µM, 72h)** | **SkBr3 IC₅₀** | **MDA-MB-231 IC₅₀** | **MCF-7 IC₅₀** |
|---|---|---|---|
| **Carboplatin** | 9 ± 1 | 161 | 89 ± 16 |
| **PMX114** | 12 ± 2 | 57, 70 | 100 |
| **PMX118** | 225, >250 | 116, 144 | 100 |

### Example 6

### Platinum cell uptake experiments in breast cancer and lung cancer cell lines by mass cytometry (CyTOF).

Cell cultures: Cancer cell lines, SkBr3, MDA-MB-231, MCF-7 and A549, which overexpress biotin receptors on their surface membranes, were each independently cultured in Dulbecco's Modified Eagle Medium (DMEM), supplemented with 10% fetal bovine serum (FBS), penicillin (100 µ/mL) and streptomycin (100 µg/mL) at 37 °C and 5% CO₂.

The platinum uptake was compared between the two cell lines each treated separately with carboplatin, PMX114 and PMX118, respectively. For MCF-7, MDA-MB-231 and SkBr3 cell lines, an additional step was performed by preincubating for 15 min with 10-100 µM biotin. The quantification was performed by means of mass cytometry. Cell singlet discrimination - staining protocol B of the mass cytometer supplier (Fluidigm) was followed.

Medium was removed from cultures and cells were pelleted at 1500 rpm. Resuspension of cells was performed in an appropriate volume of RPMI 1640 or DMEM medium to obtain a density of about 1.5-3 x 10⁶ cells per mL. Cells were fractionated and treated with different concentrations of each drug or maintained as is (control). Specifically, cells were separately treated with either 5-50 µM carboplatin, 5-50-100 µM of PMX114 or PMX118. Suspensions were incubated for 60 min at 37 °C and 5% CO₂. Suspensions used to evaluate the effect of biotin in platinum cellular uptake, were preincubated with 100 µM biotin for 15 min before the treatment with each drug. After incubation, cells were stained using Maxpar cell staining buffer (MCSB) and then fixed using 1.6% paraformaldehyde in PBS for 10 min at room temperature. Incubation with intercalator 103Rh diluted in Maxpar fix-perm buffer was performed overnight at 4 °C. Cells were washed twice with MCSB, samples were pelleted and resuspended in cell acquisition solution (CAS). A small volume of suspensions was maintained to quantify cells using Trypan blue staining. As last step, each sample was diluted with CAS and calibration beads that include metal isotopes.

### Results

Overall, platinum uptake was found to be significantly greater after treatment with PMX114 in comparison to carboplatin (Figure2).

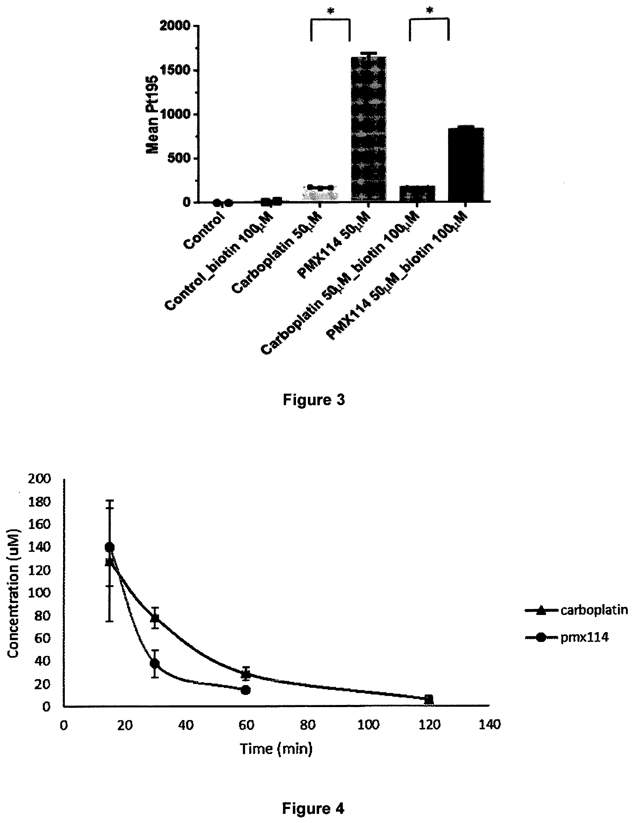

Similarly, the cellular uptake of PMX114 and PMX118 in SkBr3 cells treated separately with 50 µM of carboplatin, 50 µM of PMX114 and 50 µM of PMX118 respectively was also significantly greater than with carboplatin (Figure3). The preincubation of cells with 100 µM of biotin resulted in inhibition of platinum entry when cells were treated with PMX114.

### Example 7

### Pharmacokinetic studies in mice

Carboplatin formulated in water and PMX114 formulated in saline were intraperitoneal dosed in female C57BL/6 mice at 60 mg/kg and 75 mg/kg (equimolar doses), respectively. Samples (plasma) were collected after 15, 30, 60 and 120 min of drug administration.

All samples were analyzed by an LC-MS/MS bioanalytical methodology. Figure 4 shows the average concentration of carboplatin and PMX 114 in mouse plasma following intraperitoneal administration: carboplatin @60 mg/kg; PMX114 @75 mg/kg. The Cmax was 131±50 µM for carboplatin and 140±34 µM for PMX 114. Areas under the curve (AUCs) were 4164 µM*min for carboplatin and 2527 µM*min for PMX 114. Both compounds appear to be rapidly cleared following their administration.

### Non-Patent Citations

Azhar, A., Booker, G. W., & Polyak, S. W. (2015). Mechanisms of Biotin Transport. Biochemistry & Analytical Biochemistry, 04(04). doi:10.4172/2161-1009.1000210
Christodoulou, I., Goulielmaki, M., Kritikos, A., Zoumpourlis, P., Koliakos, G., & Zoumpourlis, V. (2022). Suitability of human mesenchymal stem cells derived from fetal umbilical cord (Wharton's jelly) as an alternative in vitro model for acute drug toxicity screening. Cells (Basel, Switzerland), 11(7), 1102. doi:10.3390/cells11071102
Dimitrijević Stojanović, M. N., Franich, A. A., Jurišević, M. M., Gajović, N. M., Arsenijević, N. N., Jovanović, I. P., ... Živković, M. D. (2022). Platinum(II) complexes with malonic acids: Synthesis, characterization, in vitro and in vivo antitumor activity and interactions with biomolecules. Journal of Inorganic Biochemistry, 231(111773), 111773. doi: 10.1 016/j.jinorgbio.2022.111773
Gholipour, N., Akhlaghi, M., Mokhtari Kheirabadi, A., Geramifar, P., & Beiki, D. (2020). Development of Ga-68 labeled, biotinylated thiosemicarbazone dextran-coated iron oxide nanoparticles as multimodal PET/MRI probe. International Journal of Biological Macromolecules, 148, 932-941. doi:10.1016/j.ijbiomac.2020.01.208
Ghosh, S. (2019). Cisplatin: The first metal based anticancer drug. Bioorganic Chemistry, 88, 102925. doi:10.1016/j.bioorg.2019.102925
Goulielmaki, M., Assimomytis, N., Rozanc, J., Taki, E., Christodoulou, I., Alexopoulos, L. G., ... Papahatjis, D. (2019). DPS-2: A novel dual MEK/ERK and PI3K/AKT pathway inhibitor with powerful ex vivo and in vivo anticancer properties. Translational Oncology, 12(7), 932-950. doi:10.1016/j.tranon.2019.04.005
Johnstone, T. C., Suntharalingam, K., & Lippard, S. J. (2016). The next generation of platinum drugs: Targeted Pt(II) agents, nanoparticle delivery, and Pt(IV) prodrugs. Chemical Reviews, 116(5), 3436-3486. doi:10.1021/acs.chemrev.5b00597
Jolley, J. N., Yanovsky, A. I., Kelland, L. R., & Nolan, K. B. (2001). Synthesis and antitumour activity of platinum(II) and platinum(IV) complexes containing ethylenediamine-derived ligands having alcohol, carboxylic acid and acetate substituents. Journal of Inorganic Biochemistry, 83(2-3), 91-100. doi:10.1016/s0162-0134(00)00190-2
Khoury, A., Deo, K. M., & Aldrich-Wright, J. R. (2020). Recent advances in platinum-based chemotherapeutics that exhibit inhibitory and targeted mechanisms of action. Journal of Inorganic Biochemistry, 207, 111070. doi:10.1016/j.jinorgbio.2020.111070
Moradell, S., Lorenzo, J., Rovira, A., Robillard, M. S., Avilés, F. X., Moreno, V., ... Llobet, A. (2003). Platinum complexes of diaminocarboxylic acids and their ethyl ester derivatives: The effect of the chelate ring size on antitumor activity and interactions with GMP and DNA. Journal of Inorganic Biochemistry, 96(4), 493-502. doi:10.1016/s0162-0134(03)00252-6
Ren, W. X., Han, J., Uhm, S., Jang, Y. J., Kang, C., Kim, J.-H., & Kim, J. S. (2015). Recent development of biotin conjugation in biological imaging, sensing, and target delivery. Chemical Communications, 51(52), 10403-10418. doi:10.1039/c5cc03075g
Rishi, G., Huang, G., & Subramaniam, V. N. (2021). Cancer: The role of iron and ferroptosis. The International Journal of Biochemistry & Cell Biology, 141, 106094. doi:10.1016/j.biocel.2021.106094
Rottenberg, S., Disler, C., & Perego, P. (2021). The rediscovery of platinum-based cancer therapy. Nature Reviews Cancer. 21, 37-50. doi:10.1038/s41568-020-00308-y
Wang, K., Liu, C., Zhu, H., Zhang, Y., Su, M., Wang, X., ... Zhu, B. (2023). Recent advances in small-molecule fluorescent probes for diagnosis of cancer cells/tissues. Coordination Chemistry Reviews, 477(214946), 214946. doi:10.1016/j.ccr.2022.214946
Wilson, J. J., & Lippard, S. J. (2014). Synthetic methods for the preparation of platinum anticancer complexes. Chemical Reviews, 114(8), 4470-4495. doi:10.1021/cr4004314
Yao, G. (1999). Asymetric total synthesis of bioactive cyclic peroxide analogs from the marine sponge Plakortis angulospiculatus: Design and synthesis of orally available cisplatin analogs (Publication No. 9916611) [Doctoral dissertation, Boston University]. ProQuest Dissertations and Theses database.
Zempleni, J., Wijeratne, S. S. K., & Hassan, Y. I. (2009). Biotin. BioFactors (Oxford, England), 35(1), 36-46. doi:10.1002/biof.8

## Claims

1. A compound of Formula (I), or a pharmaceutically acceptable salt thereof, 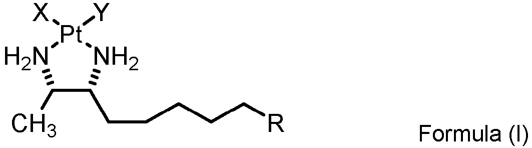 wherein
R is COOH,
X and Y are the same or different and each is selected from CI, Br, I, or,
X and Y form a ligand 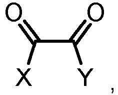
wherein each one of X and Y is O,
for use in the treatment of cancer, wherein the cancer cells overexpress biotin receptors on their cell surface membranes.

2. The compound for use according to claim 1, wherein X and Y is Cl.

3. The compound for use according to claim 1, wherein X and Y form a ligand 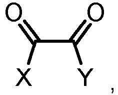 wherein each one of X and Y is O.

4. The compound for use according to claim 1 or 2, wherein the compound is

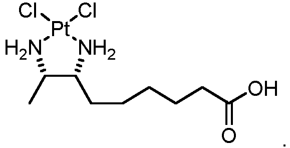

5. The compound for use according to any one of the preceding claims, wherein the cancer cells overexpress sodium-dependent multivitamin transporter (SMVT), and/or monocarboxylate transporter 1 receptors on their cell surface membranes.

6. The compound for use according to any one of the preceding claims, wherein the cancer is selected from the group consisting of breast cancer, cervical cancer, colorectal adenocarcinoma, hepatic carcinoma, human duct carcinoma, leukemia, lung cancer, mastocytoma and ovarian cancer.

7. The compound for use according to any one of the preceding claims, wherein the cancer is selected from the group consisting of breast cancer, colorectal adenocarcinoma and lung cancer.

8. The compound for use according to any one of the preceding claims, wherein the treatment includes the administration of a pharmaceutical composition comprising the compound to a subject.

9. The compound for use according to claim 8, wherein the subject is a human.

10. The compound for use according to claim 8 or 9, wherein the composition is suitable for parenteral, intramuscular, intracerebral, intravenous, intraperitoneal, subcutaneous, transdermal/intradermal, intratumoural, intrarectal, oral, or inhalation administration.

11. The compound for use according to any one of claims 8 to 10, wherein the composition has the form of powder, tablet, capsule, suppository, solution, suspension, emulsion, gel, cream, ointment, spray, or transdermal patch.

## Patentansprüche

1. Verbindung der Formel (I) oder pharmazeutisch akzeptables Salz davon, 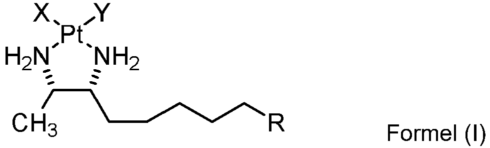 wobei
R COOH ist,
X und Y gleich oder verschieden sind und jedes unter Cl, Br, I ausgewählt ist oder
X und Y einen Liganden 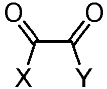
bilden,
wobei jedes von X und Y O ist,
zur Verwendung bei der Behandlung von Krebs, wobei die Krebszellen Biotinrezeptoren an ihren Zelloberflächenmembranen überexprimieren.

2. Verbindung zur Verwendung nach Anspruch 1, wobei X und Y Cl sind.

3. Verbindung zur Verwendung nach Anspruch 1, wobei X und Y einen Liganden 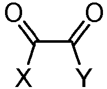 bilden,
wobei jedes von X und Y O ist.

4. Verbindung zur Verwendung nach Anspruch 1 oder 2, wobei die Verbindung 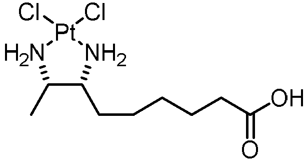 ist.

5. Verbindung zur Verwendung nach einem der vorhergehenden Ansprüche, wobei die Krebszellen natriumabhängigen Multivitamintransporter- (SMVT) und/oder Monocarboxylattransporter-1-Rezeptoren auf den Zelloberflächenmembranen überexprimieren.

6. Verbindung zur Verwendung nach einem der vorhergehenden Ansprüche, wobei der Krebs aus der Gruppe ausgewählt ist bestehend aus Brustkrebs, Gebärmutterhalskrebs, kolorektalem Adenokarzinom, Leberkarzinom, menschlichem ductalem Karzinom, Leukämie, Lungenkrebs, Mastozytom und Eierstockkrebs.

7. Verbindung zur Verwendung nach einem der vorhergehenden Ansprüche, wobei der Krebs aus der Gruppe ausgewählt ist bestehend aus Brustkrebs, kolorektalem Adenokarzinom und Lungenkrebs.

8. Verbindung zur Verwendung nach einem der vorhergehenden Ansprüche, wobei die Behandlung die Verabreichung einer pharmazeutischen Zusammensetzung, die die Verbindung umfasst, einem Subjekt umfasst.

9. Verbindung zur Verwendung nach Anspruch 8, wobei das Subjekt ein Mensch ist.

10. Verbindung zur Verwendung nach Anspruch 8 oder 9, wobei die Zusammensetzung für die parenterale, intramuskuläre, intrazerebrale, intravenöse, intraperitoneale, subkutane, transdermale/intradermale, intratumorale, intrarektale, orale oder inhalative Verabreichung geeignet ist.

11. Verbindung zur Verwendung nach einem der Ansprüche 8 bis 10, wobei die Zusammensetzung die Form von Pulver, einer Tablette, Kapsel, eines Zäpfchens, einer Lösung, Suspension, Emulsion, eines Gels, einer Creme, Salbe, eines Sprays oder transdermalen Pflasters aufweist.

## Revendications

1. Composé de formule (I), ou un sel pharmaceutiquement acceptable de celui- ci, 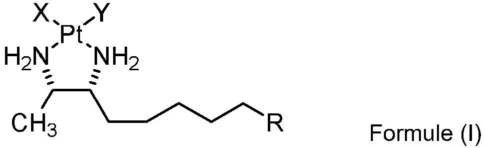 dans lequel
R représente COOH,
X et Y sont identiques ou différents et chacun est choisi parmi Cl, Br, I, ou
X et Y forment un ligand 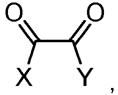
dans lequel chacun de X et Y représente O,
pour une utilisation dans le traitement d'un cancer, dans lequel le cellules cancéreuses surexpriment les récepteurs de la biotine sur leurs membranes de surface cellulaire.

2. Composé pour une utilisation selon la revendication 1, dans lequel X et Y représentent CI.

3. Composé pour une utilisation selon la revendication 1, dans lequel X et Y forment un ligand 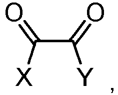 dans lequel chacun de X et Y représente O.

4. Composé pour une utilisation selon la revendication 1 ou 2, dans lequel le composé est

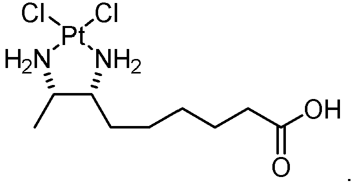

5. Composé pour une utilisation selon l'une quelconque des revendications précédentes, dans lequel les cellules cancéreuses surexpriment le transporteur de multivitamines sodium-dépendant (SMVT) et/ou les récepteurs du transporteur de monocarboxylates 1 sur la surface de leurs membranes de surface cellulaire.

6. Composé pour une utilisation selon l'une quelconque des revendications précédentes, dans lequel le cancer est choisi dans le groupe constitué par le cancer du sein, le cancer du col de l'utérus, l'adénocarcinome colorectal, le carcinome hépatocellulaire, le carcinome canalaire humain, la leucémie, le cancer du poumon, le mastocytome et le cancer de l'ovaire.

7. Composé pour une utilisation selon l'une quelconque des revendications précédentes, dans lequel le cancer est choisi dans le groupe constitué par le cancer du sein, l'adénocarcinome colorectal et le cancer du poumon.

8. Composé pour une utilisation selon l'une quelconque des revendications précédentes, dans lequel le traitement comprend l'administration à un sujet d'une composition pharmaceutique comprenant le composé.

9. Composé pour une utilisation selon la revendication 8, dans lequel le sujet est un être humain.

10. Composé pour une utilisation selon la revendication 8 ou 9, dans lequel la composition est appropriée pour une administration parentérale, intramusculaire, intracérébrale, intraveineuse, intrapéritonéale, sous-cutanée, transdermique/intradermique, intratumorale, intrarectale, orale ou par inhalation.

11. Composé pour une utilisation selon l'une quelconque des revendications 8 à 10, dans lequel la composition se présente sous forme de poudre, comprimé, capsule, suppositoire, solution, suspension, émulsion, gel, crème, pommade, spray ou timbre transdermique.
